# EUROPEAN PATENT SPECIFICATION

(11) **EP 0 395 220 B1**
(45) Date of publication and mention of the grant of the patent: **18.01.1995**
(21) Application number: 90303186.2
(22) Date of filing: 26.03.1990
(51) Int. Cl.: C07D 319/06, C09K 19/34

(54) **1,3-dioxane derivative**
1,3-Dioxanederivate
Dérivés de 1,3-dioxane

(30) Priority: 28.04.1989 JP 109649/89
(43) Date of publication of application: 31.10.1990
(73) Proprietor: SEIKO EPSON CORPORATION, Shinjuku-ku Tokyo (JP)
(72) Inventor: Obikawa, Tsuyoshi, 3-5 Owa 3-chome, Suwa-shi, Nagano-ken (JP)
(74) Representative: Sturt, Clifford Mark

(56) References cited:
- EP-A- 0 154 840
- EP-A- 0 167 912
- EP-A- 0 258 868
- US-A- 4 640 795

## Description

This invention relates to 1,3-dioxane derivatives useful as components for nematic liquid crystal compositions to be used in liquid crystal display devices.

The liquid crystal phases which are used in liquid crystal display devices are the cholesteric phase, the smectic phase and the nematic phase. There are various display methods which make use of the electro-optic effects of these liquid crystal phases. Liquid crystal display devices employing the nematic phase are widely used. The display methods employed in these devices include the dynamic scattering mode, the guest-host mode, the twisted nematic (TN) mode, the super twisted nematic (STN) mode, and the super twisted birefringence (SBE) mode. The drive methods for operating these devices include a static driving method, a time-shared drive (simple matrix drive) method, an active matrix drive method, and the two frequency drive method. Conventional liquid crystal display devices particularly using the TN mode, the STN mode and the like are advantageous in that they permit reduction in size and bulk of the liquid crystal display device, operate with a low drive voltage, consume minimum electrical power and avoid tiring users' eyes over a long period of service because they operate using incident light. As a result of these advantages liquid crystal display devices have found wide application, for example, in watches, desk top calculators, audio devices, auto-mobile instrument boards, telephone sets, and various measuring instruments. Recently, they have been used in display devices for word processors and in personal computers, and in liquid crystal television sets having a very large number of picture elements. Thus liquid crystal display devices have attracted attention as a replacement for the conventional cathode ray tube. Liquid crystal materials employed in liquid crystal display devices which are used widely, and particularly nematic liquid crystal materials employed for the TN or STN display mode, are required to have various characteristics which may be summarised as follows:-
1. The liquid crystal material should be colourless and should be stable capable of withstanding the influences of light, heat, and electrical and chemical actions.
2. The liquid crystal material should exhibit a nematic temperature in as wide a temperature range as possible.
3. The liquid crystal material should possess as low a threshold voltage (Vₜₕ) for its voltage-luminance characteristic as possible and exhibit as low a temperature dependency of the threshold voltage as permissible.
4. The liquid crystal material should enjoy a wide range of visual angle.
5. The liquid crystal material should possess a high electro-optical response speed.

There are a large number of compounds known to satisfy any one of these characteristics. However, there is no known individual liquid crystal compound which satisfies all these characteristics. In practice, therefore, to provide all the characteristics, a liquid crystal composition produced by suitable admixture of a plurality of liquid crystal compounds of different types or pseudo liquid crystal compounds, is used. The term "pseudo liquid crystal compound" as used herein refers to a compound which resembles a liquid crystal compound in molecular pattern but fails to manifest a liquid crystal phase. These pseudo liquid crystal compounds are often used in the production of liquid crystal compositions.

When a liquid crystal display device is intended for operation with a power source or for highly time shared driving, it is desirable to use as low a driving voltage as possible because driving circuitry therefor has limited resistance to voltage. Since this driving voltage is represented as Vₜₕ, it is necessary to lower Vₜₕ to the fullest possible extent. This reduction of Vₜₕ is generally attained by incorporation of a compound possessing as large a positive magnitude of dielectric anisotropy Δε as possible. Compounds heretofore used for this purpose include those of the following formulae: 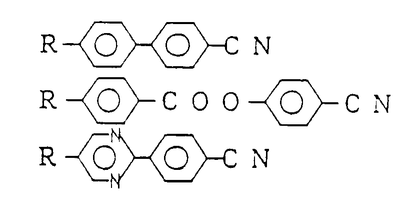
(wherein R stands for a straight chain alkyl or alkoxy group). These compounds are effective in lowering the magnitude of Vₜₕ of a liquid crystal composition in which they are incorporated but they have a large magnitude of refractive index anisotropy Δn. Incidentally, the range of visual angle mentioned above hinges on the pre-tilt of the liquid crystal molecules. It is have been known that the range of visual angle widens as the magnitude of Δn decreases. Since the compounds mentioned above have large magnitudes of Δn, they have a disadvantage that they narrow the range of visual angle.

EP-A-0154840 discloses liquid crystal compositions comprising certain 1,3-dioxane derivatives of the formula: 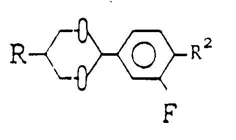
wherein R¹ is alkyl, and R² is alkyl or -CN.

The present invention, therefore, seeks to provide a 1,3-dioxane derivative useful for the production of a nematic liquid crystal composition having a low magnitude of Vₜₕ and a small magnitude of Δn.

According to one aspect of the present invention, there is provided a 1,3-dioxane derivative characterised by having the general formula: 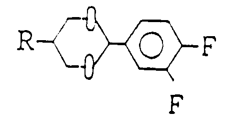
wherein R is straight chain alkyl group having 1 to 8 carbon atoms.

According to another aspect of the present invention there is provided a mixture comprising not less than 60% of the trans isomer of a 1,3-dioxane derivative according to the present invention.

According to a further aspect of the present invention there is provided a mixture comprising not more than 40% of the cis isomer of a 1-3-dioxane derivative according to the present invention.

According to a yet further aspect of the present invention, there is provided a liquid crystal composition including a derivative or mixture according to the present invention.

A 1,3-dioxane derivative according to the present invention represented by the following general formula: 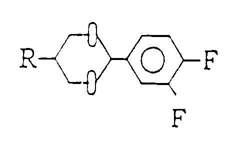
(where R is a straight chain alkyl group with 1 to 8 carbon atoms) is a novel compound and is characterised by possessing a large positive magnitude of Δε and a small magnitude of Δn. When a 1,3-dioxane derivative according to the present invention is used as a component for a nematic liquid crystal composition, therefore, there is achieved a liquid crystal display device which operates with a low drive voltage, possesses a wide range of visual angle, and permits highly time shared driving.

A 1,3-dioxane derivative according to the present invention can be synthesised as illustrated by the following Reaction Scheme:- 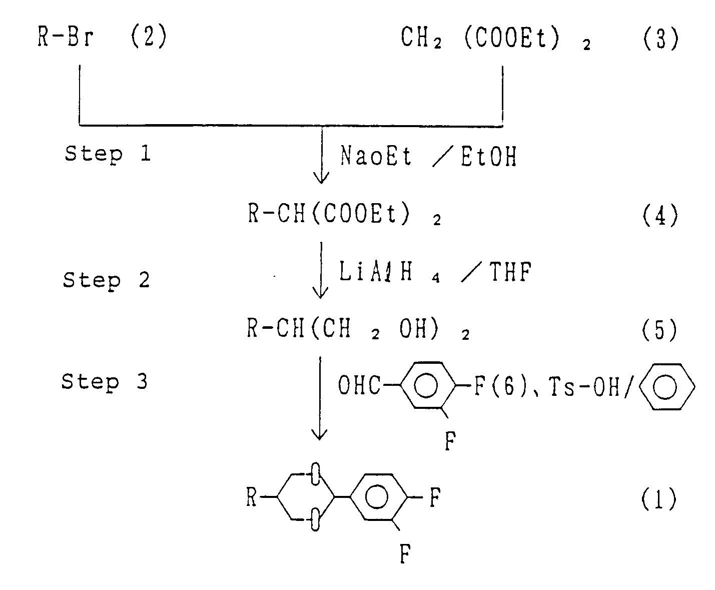
(where R is a straight chain alkyl group with 1 to 8 carbon atoms).

In Step 1, a diethyl alkylmalonate (compound (4)) is obtained by causing a bromoalkane (compound (2)) to react with diethyl malonate (compound (3)) in ethanol in the presence of sodium ethoxide.

In Step 2, a 2-alkylpropane-1,3-diol (compound (5)) is obtained by reducing the diethyl alkylmalonate with lithiumaluminium hydride in tetrahydrofuran (THF).

In Step 3, the 1,3-dioxane derivative (compound (1)) is obtained by dissolving the 2-alkylpropane-1,3-diol and 3,4-difluorobenzaldehyde in a solvent such as, for example, methylene chloride, chloroform, benezene or toluene which is capable of manifesting azeotropy with water and refluxing the resultant solution in the presence of p-toluenesulphonic acid, sulphuric acid, or a H-form cation-exchange resin as a catalyst whilst removing the water formed in a water separator.

The 1,3-dioxane derivative obtained by the above Reaction Scheme has a 1,3-dioxane ring which is a mixture of a trans isomer and a cis isomer. Though the ratio of trans isomer to cis isomer is dependent upon various factors such as reaction temperature, reaction time, and type of solvent, the proportion of the trans isomer is generally larger than that of the cis isomer. Though it would be ideal if the 1,3-dioxane derivative consisted solely of the trans isomer separation from the cis isomer is difficult. Practically, therefore, a mixture of the trans isomer and the cis isomer is used in its unmodified state. The allowable proportion of the cis isomer in the mixture will be discussed below.

The present invention will be described further, with reference to the following Examples.

### EXAMPLE 1

### Method of production of 5-pentyl-2-(3′,4′-difluorophenyl)-1,3-dioxane.

### Step 1:

In a solution of 23 g (1.0 mol) of metallic sodium in 500 cm³ of anhydrous ethanol, 160 g (1.0 mol) of diethylmalonate and 151 g (1.0 mol) of 1-bromopentane were stirred and refluxed for seven hours. The reaction product was cooled to room temperature and filtered to remove the NaBr produced. The ethanol in the filtrate was expelled by distillation and the residue of distillation was dissolved in 500 cm³ of chloroform. The resultant solution was sequentially washed with water, 5% hydrochloric acid and water in that order and heated to expel the chloroform by distillation. The residual oily substance was subjected to vacuum distillation (b.p. 103°C/3 mmHg) to obtain 193 g (0.84 mol) of diethyl pentylmalonate.

### Step 2:

In 420 cm³ of anhydrous tetrahydrofuran (THF), 35 g (0.92 mol) of lithiumaluminium hydride was dispersed. In the resultant dispersion which was kept stirred, 193 g (0.84 mol) of diethyl pentylmalonate was added dropwise at a speed sufficient to cause gentle reflux of the THF and then refluxed and stirred for three hours. The reaction product was cooled with water. To the cooled reaction product, THF containing 10% of water was added until the reaction mixture ceased to foam and 500 cm³ of concentrated hydrochloric was added. The oily layer was separated and the water layer was extracted three times from 200 cm³ of ether. The oily layer and the ether layer were jointly washed with a saturated aqueous solution of sodium chloride. The ether layer was dried with anhydrous sodium sulphate and then boiled to expel ether by distillation. The residue of distillation was subjected to vacuum distillation (b.p. 120°C/2 mmHg) to obtain 83 g (0.57 mol) of 2-pentylpropane-1,3-diol.

### Step 3:

A solution of 83 g (0.57 mol) of the 2-pentylpropane-1,3-diol, 81 g (0.57 mol) of 3,4-difluorobenzaldehyde (produced by the Aldrich Corporation), and 4.9 g (0.02 mol) of p-toluenesulphonic acid in 285 cm³ of benzene was refluxed for five hours, with the water formed being removed with a water separator. The reaction solution was washed with water, an aqueous 5% NaHCO₃ solution, and water sequentially in that order and then boiled to expel benzene by distillation. By subjecting the residual oily substance to vacuum distillation (b.p. 140°C/3.5 mmHg), there was obtain 121 g (0.45 mol) of a mixture of a trans isomer and cis isomer of 5-pentyl-2-(3′,4′-difluorophenyl)-1,3-dioxane (the ratio of trans isomer to cis isomer being 80:20). This mixture was isotropic at room temperature.

By following a similar procedure to that described above, the following mixtures were obtained:-
5-ethyl-2-(3′,4′-difluorophenyl)-1,3-dioxane (trans : cis = 70 : 30), b.p. 110°C/3 mmHg
5-propyl-2-(3′,4′-difluorophenyl)-1,3-dioxane (trans : cis = 75 : 25), b.p. 120°C/3 mmHg
5-butyl-2-(3′,4′-difluorophenyl)-1,3-dioxane (trans : cis = 77 : 23), b.p. 128°C/3 mmHg
5-hexyl-2-(3′,4′-difluorophenyl)-1,3-dioxane (trans : cis = 81 : 19), b.p. 150°C/4 mmHg
5-heptyl-2-(3′,4′-difluorophenyl)-1,3-dioxane (trans : cis = 81 : 19), b.p. 164°C/4 mmHg
5-octyl-2-(3′,4′-difluorophenyl)-1,3-dioxane (trans : cis = 80 : 20), b.p. 173°C/3 mmHg.

These mixtures were isotropic liquids at room temperature.

### EXAMPLE 2

### Effect of solvent on production of 5-pentyl-2-(3′,4′-difluorophenyl)-1,3-dioxane.

Solutions of 11.8 g (0.10 mol) of 2-pentylpropane1,3-diol, 14.2 g (0.10 mol) of 3,4-difluorobenzaldehyde, and 0.9g (0.005 mol) of p-toluenesulphonic acid obtained in Steps 1 and 2 of Example 1 respectively in methylene chloride (b.p. 40°C), chloroform (b.p. 60°C), benzene (b.p. 80°C) and toluene (b.p. 111°C) were treated by following the procedure of Step 3 of Example 1 to obtain mixtures of the cis and trans isomers of 5-pentyl-2-(3′,4′-difluorophenyl)-1,3-dioxane. The yields of these mixtures in the respective solvents and the ratios of trans isomer to cis isomer are shown in Table 1.

**Table 1**

| Solvent | b.p. (°C) | Trans : cis | Yield (%) |
|---|---|---|---|
| Methylene chloride | 40.1 | 86 : 14 | 85 |
| Chloroform | 61.2 | 84 : 16 | 87 |
| Benzene | 80.1 | 80 : 20 | 84 |
| Toluene | 110.6 | 68 : 32 | 86 |

It is to be noted from Table 1 that the mixtures comprised not less than 60% of the trans isomer even having regard to the fact that the proportion of the cis isomer formed increases as the length of the alkyl group shortens.

### EXAMPLE 3

Separation of the trans isomer alone from the mixtures obtained in Example 1 was tried. First, separation by re-crystallisation was tried but in vain. Then, separation by fractionation liquid chromotography (Hitachi Model 655) and reverse phase column (produced by Merck), C18 type measuring 5 cm in diameter and 25 cm in length and using a particulate filler 7 »m in diameter and a methanol-water (85 : 15) mixed solvent was tried. Even by this method, the separation of mixtures could not be obtained from compounds where R = C₂H₅. Table 2 shows the melting point (m.p.) of trans-5-alkyl-2-(3',4'-difluorophenyl)-1,3-dioxanes.

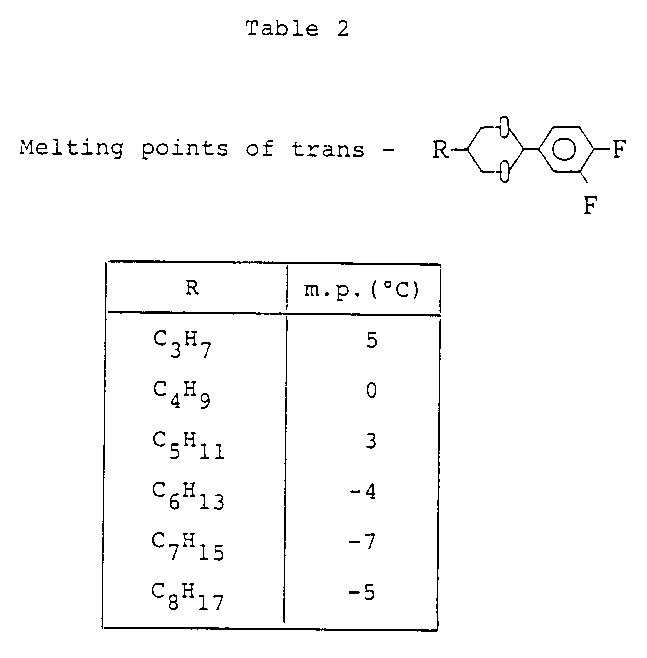

### EXAMPLE 4

The trans isomer and the cis isomer of 5-pentyl-2-(3′,4′-difluorophenyl)-1,3-dioxane separated in Example 3 were mixed in four different ratios of 20 : 80, 40 : 60, 60: 40, and 80 : 20. Liquid crystal compositions A-20, A-40, A-60 and A-80 were prepared by incorporating these mixtures respectively. Further a liquid crystal composition A-0 including solely the trans isomer and a liquid crystal composition A-100 including solely the cis isomer were also prepared. In all the liquid crystal compositions, the 5-pentyl-2-(3′,4′-difluorophenyl)-1,3-dioxane was in a fixed proportion of 10% by weight, the remainder of the liquid crystal composition being a commercially available liquid crystal composition (produced by Merck and marketed under the product code of "ZLI-1565"). These liquid crystal compositions were tested for nematic phase isotropic liquid transition point (N-I point). The results are shown in Table 3.

**Table 3**

| Composition | N-I point (°C) |
|---|---|
| A-0 | 71 |
| A-20 | 65 |
| A-40 | 52 |
| A-60 | 28 |
| A-80 | 10 |
| A-100 | Isotropic liquid |

In Example 3, separation of the trans isomer was tried using fractionation liquid chromotography. In practice this separation technique is hardly feasible from the standpoint view of both cost and production efficiency. Thus, the production of a practical liquid crystal composition requires the mixture of cis isomer and trans isomer to be used as produced. It is noted from Table 3, however, that the N-I point was low when the cis isomer content was unduly large. In practice, since the N-I point needs to be at least 50°C, the mixture desirably has a cis isomer content of not more than 40% and, consequently, a trans isomer content of not less than 60%.

### EXAMPLE 5

The following liquid crystal composition [1] and [2] were produced:- 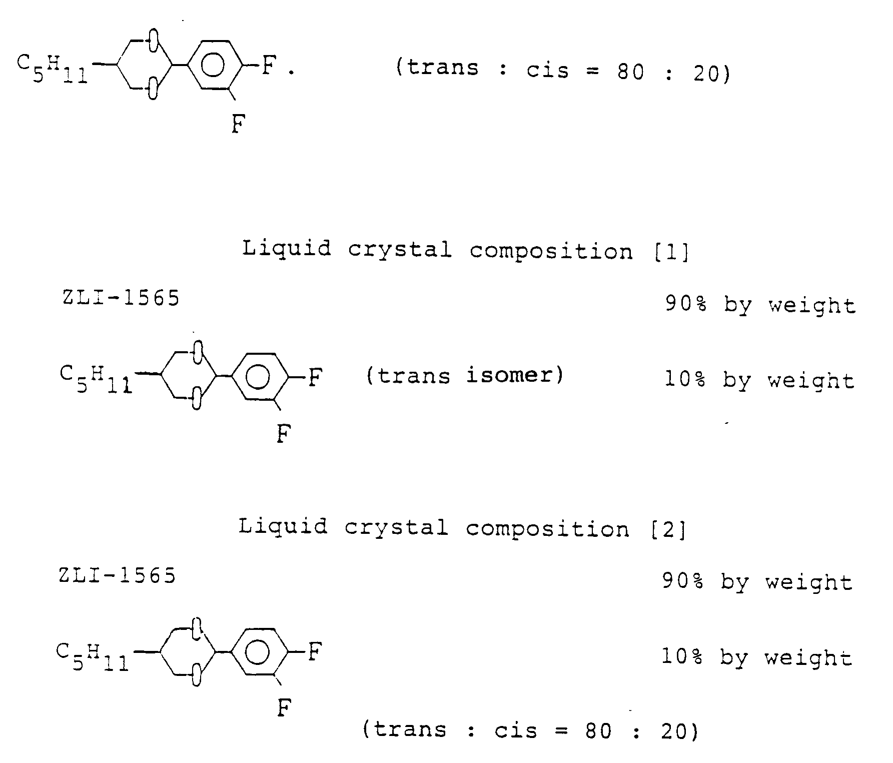
For comparison, the following liquid crystal compositions [Comparison 1] and [Comparison 2] were prepared:- 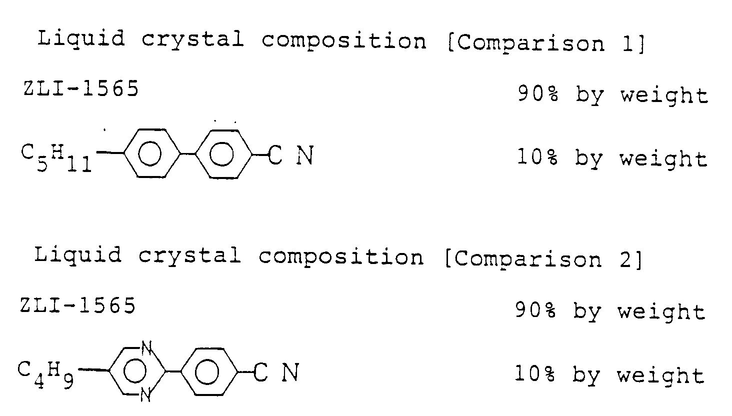
The compositions were each sealed in a TN type liquid crystal cell 8 »m in thickness and tested for voltage luminance characteristic when driven by the static driving method to determine the threshold voltage Vₜₕ (voltage required for the luminance to reach 10%). The results are shown in Table 4.

**Table 4**

| Composition | Δn | Vₜₕ (V) |
|---|---|---|
| [1] | 0.128 | 1.80 |
| [2] | 0.127 | 1.85 |
| [Comparison 1] | 0.143 | 1.80 |
| [Comparison 2] | 0.138 | 1.75 |

From the above it will be appreciated that by mixing a 1,3-dioxane derivative according to the present invention with a conventional liquid crystal composition, there is obtained a liquid crystal composition possessing small refractive index anisotropy and operating with a low driving voltage.

## Claims

1. A 1,3-dioxane derivative characterised by having the general formula: 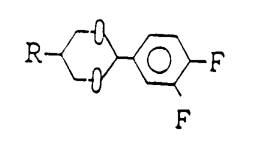 wherein R is a straight chain alkyl group having 1 to 8 carbon atoms.

2. 5-ethyl-2-(3',4'-difluorophenyl)-1,3-dioxane.

3. 5-propyl-2-(3',4'-difluorophenyl)-1,3-dioxane.

4. 5-butyl-2-(3',4'-difluorophenyl)-1,3-dioxane.

5. 5-hexyl-2-(3',4'-difluorophenyl)-1,3-dioxane.

6. 5-heptyl-2-(3',4'-difluorophenyl)-1,3-dioxane.

7. 5-octyl-2-(3',4'-difluorophenyl)-1,3-dioxane.

8. A mixture characterised by comprising not less than 60% of the trans isomer of the derivative claimed in any preceding claim.

9. A mixture characterised by comprising not more than 40% of the cis isomer of the derivative claimed in any of claims 1 to 8.

10. A liquid crystal composition including a derivative or mixture as claimed in any preceding claim.

## Patentansprüche

1. 1,3-Dioxanderivat, dadurch gekennzeichnet daß es die allgemeine Formel hat: 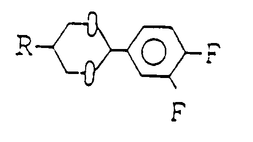 wobei R eine geradkettige Alkylgruppe mit 1 bis 8 Kohlenstoffatomen ist.

2. 5-Ethyl-2-(3',4'-Difluorphenyl)-1,3-Dioxan.

3. 5-Propyl-2-(3',4'-Difluorphenyl)-1,3-Dioxan.

4. 5-Butyl-2-(3',4'-Difluorphenyl)-1,3-Dioxan.

5. 5-Hexyl-2-(3',4'-Difluorphenyl)-1,3-Dioxan.

6. 5-Heptyl-2-(3',4'-Difluorphenyl)-1,3-Dioxan.

7. 5-Oktyl-2-(3',4'-Difluorphenyl)-1,3-Dioxan.

8. Gemisch, dadurch gekennzeichnet, daß es nicht weniger als 60% des trans-Isomers des Derivats nach einem der vorhergehenden Ansprüche hat.

9. Gemisch, dadurch gekennzeichnet, daß es nicht mehr als 40% des cis-Isomers des Derivats nach einem der Ansprüche 1 bis 8 hat.

10. Flüssigkristallzusammensetzung, umfassend ein Derivat oder Gemisch nach einem der vorhergehenden Ansprüche.

## Revendications

1. Dérivé de 1,3-dioxanne, caractérisé en ce qu'il a la formule générale suivante : 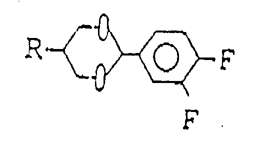 dans laquelle R est un groupe alkyle à chaîne droite ayant de 1 à 8 atomes de carbone.

2. 5-éthyl-2-(3',4'-difluorophényl)-1,3-dioxanne.

3. 5-propyl-2-(3',4'-difluorophényl)-1,3-dioxanne.

4. 5-butyl-2-(3',4'-difluorophényl)-1,3-dioxanne.

5. 5-hexyl-2-(3',4'-difluorophényl)-1,3-dioxanne.

6. 5-heptyl-2-(3',4'-difluorophényl)-1,3-dioxanne.

7. 5-octyl-2-(3',4'-difluorophényl)-1,3-dioxanne.

8. Mélange caractérisé en ce qu'il comprend au moins 60 % de l'isomère trans du dérivé selon l'une quelconque des revendications précédentes.

9. Mélange caractérisé en ce qu'il comprend au plus 40 % de l'isomère cis du dérivé selon l'une quelconque des revendications 1 à 8.

10. Composition de cristal liquide comprenant un dérivé ou un mélange selon l'une quelconque des revendications précédentes.
